(19) Europäisches Patentamt
European Patent Office
Office européen des brevets

(11) EP 0 923 549 B1

(12) **FASCICULE DE BREVET EUROPEEN**

(45) Date de publication et mention de la délivrance du brevet:
**15.05.2002 Bulletin 2002/20**

(21) Numéro de dépôt: **97928341.3**

(22) Date de dépôt: **12.06.1997**

(51) Int Cl.[7]: **C07D 209/16**, A61K 31/40

(86) Numéro de dépôt international:
**PCT/FR97/01053**

(87) Numéro de publication internationale:
**WO 97/48680 (24.12.1997 Gazette 1997/55)**

(54) **SEL DE METHANESULFONATE D'UNE ARYLPIPERAZINE DERIVEE DE TRYPTAMINE ET SES SOLVATES POUR USAGE PHARMACEUTIQUE**

METHANSULFONATSALZ EINES ARYLPIPERAZINDERIVAT VON TRYPTAMIN UND SEINE SOLVATE ZUR PHARMAZEUTISCHE VERWENDUNG

METHANESULPHONATE SALT OF AN ARYLPIPERAZINE DERIVED FROM TRYPTAMINE AND ITS SOLVATES FOR PHARMACEUTICAL USE

(84) Etats contractants désignés:
**AT BE CH DE DK ES FI FR GB GR IE IT LI LU MC NL PT SE**

(30) Priorité: **17.06.1996 FR 9607491**

(43) Date de publication de la demande:
**23.06.1999 Bulletin 1999/25**

(73) Titulaire: **PIERRE FABRE MEDICAMENT**
**92100 Boulogne-Billancourt (FR)**

(72) Inventeurs:
- **HALAZY, Serge**
  **F-81090 Lagarrigue (FR)**
- **PEREZ, Michel**
  **F-81100 Castres (FR)**

(74) Mandataire: **Ahner, Francis et al**
**Cabinet Régimbeau**
**20, rue de Chazelles**
**75847 Paris cedex 17 (FR)**

(56) Documents cités:
**EP-A- 0 490 689**
**WO-A-95/14004**
**GB-A- 2 162 522**
**GB-A- 2 185 020**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 923 549 B1

## Description

La présente invention a pour objet un sel particulier d'un agent pharmaceutique actif. Plus particulièrement, l'invention se rapporte au sel de méthanesulfonate d'une arylpipérazide dérivée de tryptamine qui agit au niveau des récepteurs de la sérotonine (5-HT), plus précisément comme agoniste sélectif des récepteurs $5\text{-}HT_{1D/1B}$. Ce composé trouve dès lors son utilité pour le traitement de conditions pathologiques pour lesquelles un agoniste sélectif de ces récepteurs est indiqué.

Les agonistes des récepteurs $5\text{-}HT_{1D/1B}$ (selon la nomenclature récente proposée dans TiPS, 17, 103, 1996) qui démontrent une activité vasoconstrictrice sélective ont récemment été décrits comme utiles dans le traitement de la migraine (cf. par exemple A. Doenicke et Coll. The Lancet, 1, 1309, 1988). Le sel de la présente invention, qui montre une activité agoniste puissante au niveau des récepteurs $5\text{-}HT_{1D/1B}$ est dès lors particulièrement utile pour le traitement tant curatif que préventif de la douleur, des crises de migraine classique (avec aura), commune (sans aura) et des désordres associés ou proches tels que les désordres vasospastiques, l'algie vasculaire de la face, les céphalées chroniques vasculaires.

La demande de brevet PCT FR 9401343 (WO-9514004) décrit une classe d'arylpipérazines dérivées d'indole comme agonistes sélectifs et efficaces des récepteurs $5\text{-}HT_{1D/1B}$ et en conséquence comme particulièrement utiles pour le traitement de la migraine et désordres associés.

La présente invention concerne le sel de mésylate (ou méthanesulfonate) du 4-(4-{2-[3-(2-amino-éthyl)-1H-indol-5-yloxy]-acétyl}-pipérazin-1-yl)-benzonitrile de formule (I) :

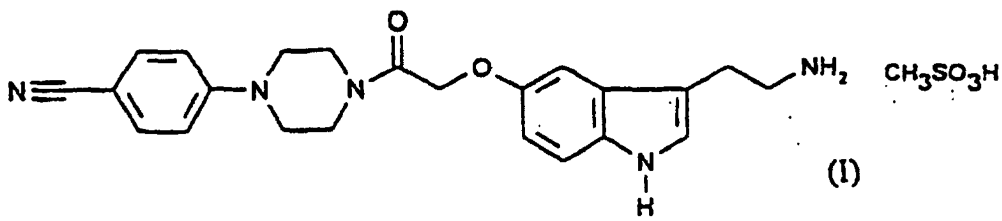

(I)

ainsi que ses solvates (en particulier ses hydrates) acceptables pour l'usage thérapeutique.

Les sels pharmaceutiquement acceptables du 4-(4-{2-[3-(2-amino-éthyl)-1H-indol-5-yloxy]-acétyl}-pipérazin-1-yl)-benzonitrile font partie, d'une manière générale, de la demande de brevet WO-9514004. En fait, les sels de chlochydrate, bromhydrate, sulfate, maléate et fumarate sont spécifiquement désignés dans la demande WO-9514004. Toutefois, en aucun cas, cette demande de brevet ne décrit, ni ne suggère le sel particulier de formule (I) faisant partie de la présente invention.

Le sel de formule (I), ci-dessus, de façon tout à fait inattendue, possède des avantages intéressants en particulier pour son utilisation comme agent thérapeutique. Par exemple, le composé de formule (I) présente une solubilité nettement supérieure aux autres sels du 4-(4-{2-[3-(2-amino-éthyl)-1H-indol-5-yloxy]-acétyl}-pipérazin-1-yl)-benzonitrile revendiqués spécifiquement dans la demande WO-9514004.

Un autre aspect de la présente invention a pour objet les compositions à usage pharmaceutique comprenant le sel de méthanesulfonate (1) en association avec un ou plusieurs véhicules pharmaceutiques acceptables pour l'usage thérapeutique.

La présente invention concerne également les médicaments constitués par au moins un composé de formule (I) à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale, nasale ou toute autre voie d'administration.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que des diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables,

par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée ; elles sont généralement comprises entre 0,0001 g et 1 g (de préférence comprises entre 0,0005 g et 0,25 g) par jour de préférence par voie orale ou nasale pour un adulte avec des doses unitaires allant de 0,05 mg à 500 mg de substance active, de préférence de 0,5 mg à 50 mg.

D'une façon générale, le médecin déterminera la posologie appropriée en fonction de l'âge, du poids et de tous les autres facteurs propres au sujet à traiter. Les exemples suivants illustrent des compositions selon l'invention [dans ces exemples, le terme "composant actif" désigne le composé de formule (I) défini comme précédemment :

<u>Comprimés</u>

On peut les préparer par compression directe ou en passant par une granulation au mouillé. Le mode opératoire par compression directe est préféré mais il peut ne pas convenir dans tous les cas selon les doses du composant actif.

*A - Par compression directe*

| | mg pour un comprimé |
|---|---|
| composant actif | 10,0 |
| cellulose microcristalline B.P.C. | 89,5 |
| stéarate de magnésium | 0.5 |
| | 100,0 |

On passe le composant actif au travers d'un tamis à ouverture de maille de 250 μm de côté, on mélange avec les excipients et on comprime à l'aide de poinçons de 6,0 mm. On peut préparer des comprimés présentant d'autres résistances mécaniques en modifiant le poids de compression avec utilisation de poinçons appropriés.

*B - Granulation au mouillé*

| | mg pour un comprimé |
|---|---|
| composant actif | 10,0 |
| lactose Codex | 74,5 |
| amidon Codex | 10,0 |
| amidon de maïs prégélatinisé Codex | 5,0 |
| stéarate de magnésium | 0.5 |
| Poids à la compression | 100,0 |

On fait passer le composant actif au travers d'un tamis à ouverture de maille de 250 μm et on mélange avec le lactose, l'amidon et l'amidon prégélatinisé. On humidifie les poudres mélangées par de l'eau purifiée, on met à l'état de granulés, on sèche, on tamise et on mélange avec le stéarate de magnésium. Les granulés lubrifiés sont mis en comprimés comme pour les formules par compression directe. On peut appliquer sur les comprimés une pellicule de

revêtement au moyen de matières filmogènes appropriées, par exemple la méthylcellulose ou l'hydroxy-propyl-méthyl-cellulose, selon des techniques classiques. On peut également revêtir les comprimés de sucre.

Capsules

| | mg pour une capsule |
|---|---|
| composant actif | 10,0 |
| *amidon 1500 | 89,5 |
| stéarate de magnésium Codex | 0.5 |
| Poids de remplissage | 100,0 |

*une forme d'amidon directement compressible provenant de la firme Colorcon Ltd, Orpington, Kent, Royaume Uni.

On fait passer le composant actif au travers d'un tamis à ouverture de maille de 250 µm et on mélange avec les autres substances. On introduit le mélangé dans des capsules de gélatine dure n°2 sur une machine à remplir appropriée. On peut préparer d'autres unités de dosage en modifiant le poids de remplissage et, lorsque c'est nécessaire, en changeant la dimension de la capsule.

Sirop

| | | mg par dose de 5 ml |
|---|---|---|
| composant actif | | 10,0 |
| saccharose Codex | | 2750,0 |
| glycérine Codex | | 500,0 |
| tampon | ) | |
| arôme | ) | |
| colorant | ) | q.s. |
| préservateur | ) | |
| eau distillée | | 5,0 |

On dissout le composant actif, le tampon, l'arôme, le colorant et le préservateur dans une partie de l'eau et on ajoute la glycérine. On chauffe le restant de l'eau à 80°C et on y dissout le saccharose puis on refroidit. On combine les deux solutions, on règle le volume et on mélange. Le sirop obtenu est clarifié par filtration.

Suppositoires

| | |
|---|---|
| Composant actif | 10,0 mg |
| *Witepsol H15 complément à | 1,0 g |

*Marque commercialisée pour Adeps Solidus de la Pharmacopée Européenne.

On prépare une suspension du composant actif dans le Witepsol H15 et on l'introduit dans une machine appropriée avec moules à suppositoires de 1g.

Liquide pour administration par injection intraveineuse

| | g/l |
|---|---|
| composant actif | 2,0 |
| eau pour injection Codex complément à | 1000,0 |

On peut ajouter du chlorure de sodium pour régler la tonicité de la solution et régler le pH à la stabilité maximale et/ou pour faciliter la dissolution du composant actif au moyen d'un acide ou d'un alcali dilué ou en ajoutant des sels tampons appropriés. On prépare la solution, on la clarifie et on l'introduit dans des ampoules de dimension appropriée qu'on scelle par fusion du verre. On peut également stériliser le liquide pour injection par chauffage à l'autoclave selon l'un des cycles acceptables. On peut également stériliser la solution par filtration et introduire en ampoule stérile dans des conditions aseptiques. La solution peut être introduite dans les ampoules en atmosphère gazeuse.

Cartouches pour inhalation

| | g/cartouche |
|---|---|
| composant actif micronisé | 1,0 |
| lactose Codex | 39,0 |

Le composant actif est micronisé dans un broyeur à énergie de fluide et mis à l'état de fines particules avant mélange avec du lactose pour comprimés dans un mélangeur à haute énergie. Le mélange pulvérulent est introduit en capsules de gélatine dure n°3 sur une machine à encapsuler appropriée. Le contenu des cartouches est administré à l'aide d'un inhalateur à poudre.

L'administration d'un anti-migraineux par voie orale peut rencontrer certains problèmes dans la mesure où, la migraine (ainsi que d'autres conditions pathologiques proches ou associées) est parfois accompagnée de nausées et vomissements qui rendent difficile la tolérance d'une administration de médicaments par voie orale. En conséquence, l'administration d'un anti-migraineux par voie nasale constitue une alternative particulièrement intéressante.

Le sel de formule (I) s'avère, de façon surprenante, beaucoup plus soluble dans l'eau (cf. table 1) que les autres sels dérivés de la même base aminée et décrits dans la demande de brevet WO-9514004.

Table 1

| Comparaison de la solubilité dans l'eau de différents sels du 4-(4-{2-[3-(2-amino-éthyl)-1H-indol-5-yloxy]-acétyl}-pipérazin-1-yl)-benzonitrile | |
|---|---|
| SEL | SOLUBILITE* |
| Méthanesulfonate (composé 1) | 4,0 |
| Chlorhydrate | 0,87 |
| Sulfate | <1 |
| Maléate | <1 |
| Fumarate | <1 |

* exprimée en mg de base par ml d'eau

Le composé (I) de la présente invention peut dès lors être particulièrement apprécié pour son utilisation thérapeutique sous forme de solution et en particulier pour son usage via une administration par voie nasale ou orale. La présente invention comprend donc une composition pharmaceutique adaptée pour l'administration par voie nasale ou orale, composition qui comprend le sel de méthanesulfonate de formule (I) en association avec un ou plusieurs véhicules acceptables pour l'usage thérapeutique.

Les solutions utilisées pour l'administration du composé de formule (I) sont généralement des solutions aqueuses, préparées à partir de l'eau elle-même (par exemple de l'eau stérile) ou à partir d'eau et d'un co-solvant pharmaceutiquement acceptable tel que par exemple l'éthanol, le propylèneglycol ou des polyéthylèneglycols. De telles solutions peuvent éventuellement contenir d'autres excipients tels que des agents préservatifs (par exemple le chlorure

de benzalkonium ou le phénéthylalcool), des agents tampons, des agents propices à ajuster la pression osmotique (par exemple NaCl), des agents aromatisants (tels que par exemple le menthol, l'eucalyptol, ou le camphre), des agents édulcorants (par exemple la saccharine ou l'aspartam).

Les solutions pour administration par voie nasale sont appliquées directement dans la cavité intranasale par les moyens conventionnels en utilisant par exemple un compte-gouttes, une pipette ou un spray. La formulation peut être envisagée pour administration en dose unique ou sous la forme de doses répétées. L'administration intranasale peut également être réalisée au moyen d'une formulation de type aérosol dans laquelle le composé de formule (I) est disponible dans un flacon sous pression avec un gaz propulseur tel qu'un chlorofluorocarbone (CFC) ou le dioxyde de carbone.

Une composition pharmaceutique appropriée à l'administration par voie nasale, particulièrement appréciée et faisant partie de la présente invention contient le sel de formule (I) sous forme d'une solution aqueuse,

Les solutions aqueuses du sel (I) revendiqué dans la présente invention pour administration par voie intranasale auront préférentiellement un pH compris entre 4 et 8 et plus particulièrement entre 5 et 7. Les solutions aqueuses du sel de méthanesulfonate de formule (I) sont préparées par dissolution du sel de formule (I) dans l'eau. Une méthode alternative de préparation de solutions aqueuses du sel de méthanesulfonate de formule (I) consiste à mélanger un équivalent molaire de 4-(4-{2-[3-(2-amino-éthyl)-1H-indol-5-yloxy]-acétyl}-pipérazin-1-yl)-benzonitrile avec 1,0 à 1,2 équivalent molaire d'acide méthanesulfonique, de préférence 1.0 équivalent dans l'eau.

Pour l'administration intranasale, les solutions aquesuses du sel de formule (I) selon la présente invention contiendront préférentiellement le sel à une concentration comprise entre 1 mg/ml et 4 mg/ml, et préférentiellement de 1,5 mg/ml à 4 mg/ml.

La présente invention comprend également un procédé de fabrication du sel de mésylate de formule (I) comme défini précédemment qui consiste à traiter un intermédiaire de formule (II)

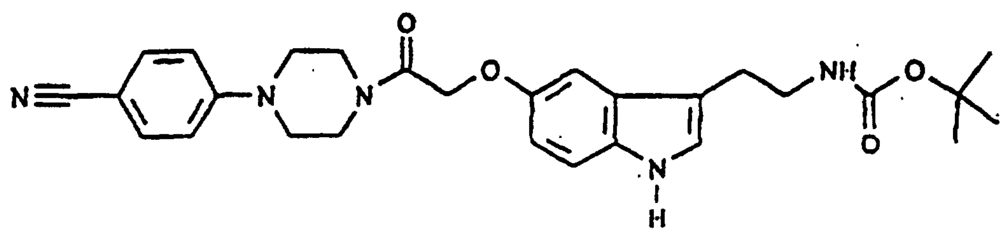

(II)

avec l'acide méthanesulfonique généralement à une température comprise entre 0°C et 80°C, soit dans l'eau, soit dans un solvant organique tel que le chloroforme, le dichlorométhane, le dichloroéthane, le THF, l'éther éthylique, le dioxanne, le DMF, le nitrométhane ou encore dans un mélange eau/THF, eau/dioxanne, eau/acétone ou eau/DMF. Une méthode particulièrement appréciée pour préparer le sel de méthanesulfonate de formule (I) consiste à traiter le dérivé N-t-butoxycarbonyl de formule (II) avec 1 à 3 équivalents d'acide méthanesulfonique dans le chloroforme à une température comprise entre 15 et 30°C.

Le composé de formule (II) décrit précédemment est préparé par diverses méthodes et techniques bien connues de l'homme de l'art pour préparer ce type de dérivés comme par exemple celles décrites dans la demande de brevet WO-9514004. Une méthode particulièrement appréciée pour la préparation du composé de formule (II) consiste à condenser l'arylpipérazine de formule (III)

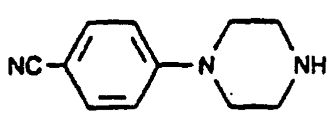

(III)

avec l'acide carboxylique de formule (IV) ou l'un de ses dérivés appropriés pour la condensation avec une amine dans le but de préparer une amide

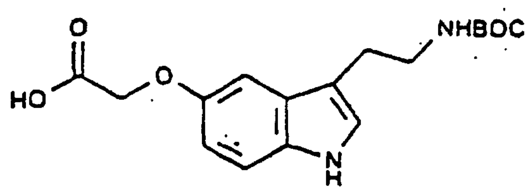

(IV)

La condensation de l'acide carboxylique de formule (IV) avec l'arylpipérazine de formule (III) sera réalisée par les méthodes et techniques bien connues de l'homme de métier pour la préparation d'amides à partir d'amines et d'acides carboxyliques, méthodes qui comprennent la mise en oeuvre de dérivés de l'acide carboxylique de formule (IV) tels que le chlorure d'acide correspondant. Une méthode particulièrement appréciée pour la préparation de l'intermédiaire de formule (II) consiste à activer l'intermédiaire (IV) par traitement avec le chloroformate d'éthyle, dans un solvant organique tel que le dichlorométhane, à une température comprise entre - 5°C et - 20°C et en présence d'une base tel que la N-méthylmorpholine. La réaction avec la pipérazine (III) à une température allant de - 15°C à 25°C conduit à l'intermédiaire (II) avec un rendement de 82 %.

La procédure suivante illustre la méthode de préparation du sel de formule (I) à partir de l'intermédiaire (II):

L'intermédiaire (II) (1,3 g ; 2,58 mmol) en solution dans le dichlorométhane (50 ml) est traité, à température ambiante, par l'acide méthanesulfonique (0,335 ml ; 5,16 mmol). Le mélange est agité pendant 24 heures puis le précipité blanc formé est filtré sur fritté et lavé par un mélange dichlorométhane/éthanol : 2/l (20 ml) et à l'éther (30 ml). Les cristaux ainsi obtenus sont séchés sous vide pour conduire au composé (I) (1,12 g ; 87 %).

| Analyse élémentaire ($C_{24}H_{29}N_5O_5S$, 0,60$H_2O$) | | | |
|---|---|---|---|
| % Calculés | C : 56,08 ; | H : 6,00 ; | N : 13,63 |
| % Trouvés | C : 55,91, | H : 5,80 ; | N : 13,63 |

RMN $^1$H, DMSO-d6 (ppm) : 2,33 s, 3H ; 2,90-3,10 m, 4H ; 3,45 m, 4H ; 3,63 m, 4H ; 4,80 s, 2H ; 6,79 dd, 1H ; 7,02 d, 2H ; 7,09 d, 1H ; 7,19 d, 1H ; 7,25 d, 1H ; 7,60 d, 2H ; 7,75 large s, 3H ; 10,83 s, 1H

Point de fusion : 142°C.

## Revendications

1. Le sel de méthanesulfonate du 4-(4-{2-[3-(2-amino-éthyl)-1H-indol-5-yloxy]-acétyl}-pipérazin-1-yl)-benzonitrile de formule (I)

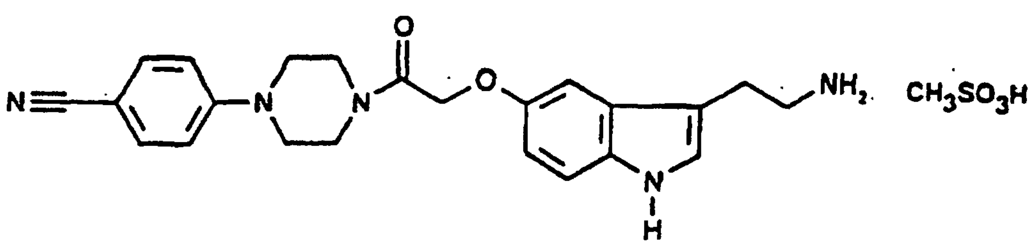

(I)

ainsi que ses hydrates ou solvates acceptables pour l'usage pharmaceutique.

2. Compositions pharmaceutiques comprenant, à titre d'ingrédient actif, le composé de formule (I) selon la revendication 1, en combinaison avec un véhicule pharmaceutique acceptable pour l'usage thérapeutique.

3. Compositions pharmaceutiques adaptées pour l'administration nasale ou orale comprenant à titre d'ingrédient actif le composé de formule (I) selon la revendication 1 en combinaison avec un véhicule pharmaceutique acceptable pour l'usage thérapeutique.

4. Composition pharmaceutique selon l'une des revendications 2 ou 3 dans laquelle le composé de formule (I) selon la revendication 1 est présenté sous forme d'une solution aqueuse.

5. Composition pharmaceutique selon la revendication 4 contenant le sel de formule (I) à une concentration comprise entre 1 et 4 mg/ml.

6. Procédé de préparation du sel de formule (I) selon la revendication 1 qui consiste à traiter un dérivé de formule (II)

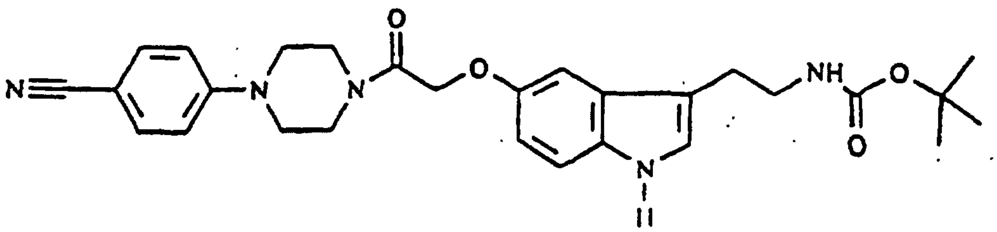

(II)

avec l'acide méthanesulfonique dans un solvant tel que le chloroforme ou le dichlorométhane.

7. L'utilisation du sel de formule (I) selon la revendication 1 pour la préparation de médicaments pour le traitement tant préventif que curatif de la douleur, des crises de migraine, de l'algie vasculaire de la face et des céphalées chroniques.

## Claims

1. Methanesulphonate salt of 4-(4-{2-[3-(2-aminoethyl)-1H-indol-5-yloxy]acetyl}piperazin-1-yl)benzonitrile of formula (I)

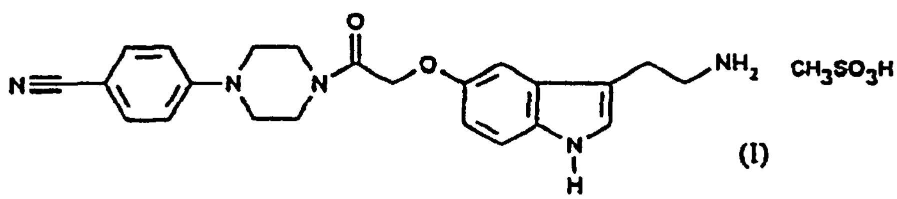

(I)

and its hydrates or solvates which are acceptable for the pharmaceutical use.

2. Pharmaceutical compositions comprising, as active ingredient, the compound of formula (I) according to Claim 1 in combination with a pharmaceutical vehicle which is acceptable for the therapeutic use.

3. Pharmaceutical compositions which are suited to nasal or oral administration comprising, as active ingredient, the compound of formula (I) according to Claim 1 in combination with a pharmaceutical vehicle which is acceptable for the therapeutic use.

4. Pharmaceutical composition according to either of Claims 2 and 3, in which the compound of formula (I) according to Claim 1 is present in the form of an aqueous solution.

5. Pharmaceutical composition according to Claim 4 comprising the salt of formula (I) at a concentration of between 1 and 4 mg/ml.

6. Process for the preparation of the salt of formula (I) according to Claim 1, which consists in treating a derivative of formula (II)

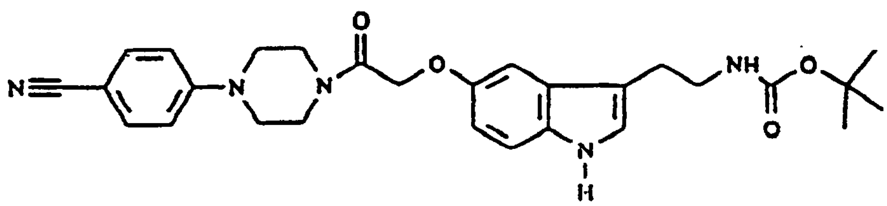

(II)

with methanesulphonic acid in a solvent such as chloroform or dichloromethane.

7. Use of the salt of formula (I) according to Claim 1 in the preparation of medicaments for the treatment, both preventive and curative, of pain, migraine attacks, facial vascular pain and chronic headaches.

**Patentansprüche**

1. Methansulfonatsalz von 4-(4-{2-[3-(2-Aminoethyl)-1H-indol-5-yloxy]acetyl}-piperazin-1-yl)benzonitril der Formel (I)

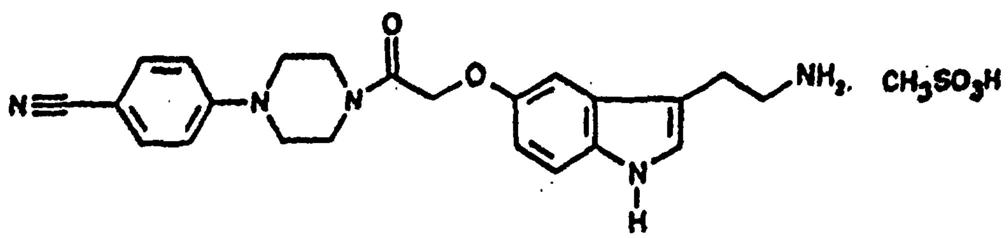

sowie seiner für die pharmazeutische Verwendung annehmbaren Hydrate oder Solvate.

2. Pharmazeutische Zusammensetzungen, die als Wirkstoff die Verbindung der Formel (I) nach Anspruch 1 in Kombination mit einem pharmazeutischen, für die therapeutische Verwendung annehmbaren Vehikel umfassen.

3. Pharmazeutische Zusammensetzungen, die für die nasale oder orale Verabreichung angepasst sind und als Wirkstoff die Verbindung der Formel (I) nach Anspruch 1 in Kombination mit einem pharmazeutischen, für die therapeutische Verwendung annehmbaren Vehikel umfassen.

4. Pharmazeutische Zusammensetzung nach einem der Ansprüche 2 oder 3, in welcher die Verbindung der Formel (I) nach Anspruch 1 in Form einer wässrigen Lösung vorliegt.

5. Pharmazeutische Zusammensetzung nach Anspruch 4, welche das Salz der Formel (I) bei einer Konzentration von 1 bis 4 mg/ml enthält.

6. Verfahren zur Herstellung des Salzes der Formel (I) nach Anspruch 1, welches darin besteht, ein Derivat der Formel (II)

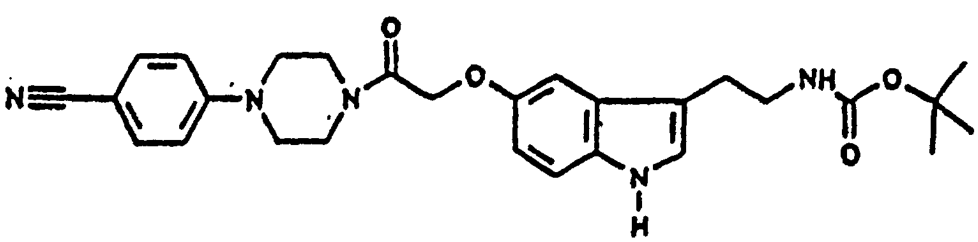

mit Methansulfonsäure in einem Lösungsmittel wie Chloroform oder Dichlormethan zu behandeln.

**7.** Verwendung des Salzes der Formel (I) nach Anspruch 1 für die Herstellung eines Medikamentes sowohl für die vorbeugende als auch die hellende Behandlung von Schmerz, Migräneanfällen, Gefäßschmerzen des Gesichts und chronischen Kopfschmerzen.